(19) **Europäisches Patentamt**

**European Patent Office**

**Office européen des brevets**

(11) **EP 0 841 897 B2**

(12) **NOUVEAU FASCICULE DE BREVET EUROPEEN**

(45) Date de publication et mention de la décision concernant l'opposition:
**30.06.2004 Bulletin 2004/27**

(45) Mention de la délivrance du brevet:
**23.05.2001 Bulletin 2001/21**

(21) Numéro de dépôt: **96927728.4**

(22) Date de dépôt: **02.08.1996**

(51) Int Cl.$^7$: **A61K 7/48**

(86) Numéro de dépôt international:
**PCT/FR1996/001229**

(87) Numéro de publication internationale:
**WO 1997/005856 (20.02.1997 Gazette 1997/09)**

(54) **GEL A ACTIVITE PHYSIQUE ANTIMICROBIENNE POUR PRODUITS COSMETIQUES**

PHYSIKALISCH AKTIVES ANTIMIKROBIELLES GEL FUER KOSMETISCHE ERZEUGNISSE

PHYSICALLY ACTIVE ANTIMICROBIAL GEL FOR COSMETIC PRODUCTS

(84) Etats contractants désignés:
**AT BE CH DE DK ES FI FR GB GR IE IT LI NL PT SE**

(30) Priorité: **04.08.1995 FR 9509512**

(43) Date de publication de la demande:
**20.05.1998 Bulletin 1998/21**

(73) Titulaire: **SEDERMA S.A.**
**78610 Le Perray-en-Yvelines Cédex (FR)**

(72) Inventeur: **GREFF, Daniel**
**F-78490 Mère (FR)**

(74) Mandataire: **Laget, Jean-Loup**
**Cabinet Loyer,**
**78, avenue Raymond Poincaré**
**75116 Paris (FR)**

(56) Documents cités:
**EP-A- 0 499 304        WO-A-94/21234**
**WO-A-95/35093        FR-A- 2 682 296**
**GB-A- 2 085 020**

**Description**

L'invention concerne un gel à activité physique antimicrobienne pour produits cosmétiques ou dermopharmaceutiques.

La plupart des produits cosmétiques et dermopharmaceutiques destinés à l'application topique - et quelle que soit la forme galénique : émulsion H/E, E/H, lait, lotion, gel, solution - contiennent une ou plusieurs substances microbicides dans leur formule. Les raisons en sont évidentes : les matières premières utilisées pour la fabrication de ces produits ne sont que rarement parfaitement stériles, les produits finis sont trop fragiles (parfum, actifs biologiques, vitamines) pour supporter une stérilisation après conditionnement. Il faut donc protéger le produit contre toute contamination microbienne qui nuirait à la santé de l'utilisateur ou à l'aspect esthétique du produit. Ces contaminations peuvent provenir du procédé de fabrication (y compris les matières premières utilisées), d'un emballage non hermétique et surtout après ouverture, de l'environnement et de l'usager lui-même.

Contrairement aux produits alimentaires ou aux produits pharmaceutiques, les produits cosmétiques ne sont pas obligés de porter une date de limite de consommation ; ils peuvent et doivent donc être stables et en parfait état - y compris microbiologiquement - pendant très longtemps.

Les conservateurs chimiques utilisés dans ces produits remplissent ce rôle de protection. Le choix des substances antimicrobiennes ou molécules biocides pouvant être employées est strictement réglementé par les différentes législations des pays européens, américains et asiatiques.

Puisque tout conservateur doit, pour être efficace, agir par voie chimique en détruisant soit la paroi cellulaire, soit les mécanismes biochimiques des cellules microbiennes, il n'est pas surprenant que ces mêmes molécules biocides aient parfois un effet nocif sur les cellules humaines avec lesquelles elles entrent en contact. Il est un fait qu'avec l'augmentation de la consommation de produits cosmétiques et dermopharmaceutiques on observe une augmentation parallèle de cas d'intolérance (irritation, allergie) vis à vis de ces produits. Les analyses effectuées par différents dermatologues, pharmaciens, ou autorités sanitaires, révèlent qu'un certain nombre de ces incidents peuvent être attribués aux conservateurs contenus dans les produits.

En plus, la tendance écologique de notre époque renforce le désir de l'industrie cosmétique d'offrir des produits "naturels", "doux", ou "hypoallergisants". On cherche donc le moyen de protéger les crèmes sans y incorporer des conservateurs.

Formuler des produits cosmétiques sans conservateurs n'est cependant pas facile. C'est actuellement possible en utilisant des matières premières sélectionnées, stériles et en assurant la fabrication dans des conditions strictes d'hygiène aseptique (conditionnement sous flux laminaire dans des contenants stériles). Il reste le risque de contamination lors de l'utilisation. Seul le choix d'un emballage spécial, conçu pour libérer la dose d'utilisation au moment de l'application, et évitant toute entrée d'air et de germes vers l'intérieur, peut répondre à ces exigences.

Tout cela limite donc la diversité des gammes de produits cosmétiques que l'on peut préparer sans conservateur, et surtout augmente considérablement le coût de fabrication, de conditionnement et d'emballage.

Le brevet FR 2 682 296 a proposé une méthode de conservation non-chimique qui est basée sur l'utilisation de gels du type glyceryl poly(méth)acrylate, dont la propriété est d'exercer un fort effet osmotique sur son environnement, ce qui permet d'inactiver les microorganismes introduits dans une préparation cosmétique en les privant d'eau. Des tests microbiologiques de surcontamination (par exemple, l'inoculation avec $10^6$ germes/gramme d'u ne crème contenant 40 % du gel en question est maîtrisée en 7 jours seulement par la présence du gel dans la formule, en l'absence de tout conservateur chimique) permettent de démontrer cette efficacité antimicrobienne du gel. La méthode décrite dans ce brevet s'est avérée applicable dans la pratique, mais elle possède des inconvénients : la quantité de gel nécessaire dans une formulation cosmétique est très élevée (minimum 40 % dans les meilleurs cas ; souvent il faut incorporer le gel dans la proportion de 50, voire 60 %). Cela limite le champ d'application, puisque le formulateur est soumis à des contraintes dans sa liberté d'adapter la texture, le toucher des produits. Un autre inconvénient s'est manifesté au cours des essais successifs : la protection des produits finis contre une contamination par certaines moisissures (ex. Aspergillus niger) est très difficile à assurer, ce germe ne nécessitant que peu d'eau libre pour son développement ; il est donc moins sensible à la privation d'eau assurée par le gel.

WO 94/21234 a décrit des compositions cosmétiques utilisables dans le domaine de la parfumerie. Ce sont soit des gels, soit des émulsions, ou des liquides, contenant comme préproduits de base un éther-alcool et un polymère acrylique dispersé dans ce solvant, en vue de la solubilisation des parfums, et non de l'inhibition de l'activité microbienne. Des polyols [PPG + butylèneglycol] peuvent être éventuellement présents en tant qu'ingrédients cosmétiques appropriés et non comme agents antimicrobiens. Il est en effet nécessaire d'ajouter des germicides, tels que le Triclosan au gel lui-même.

Un but de l'invention est de proposer un gel qui présente une efficacité accrue dans la conservation des produits cosmétiques, en particulier contre les moisissures susceptibles de se développer, c'est-à-dire un gel présentant des propriétés osmotiques améliorées.

Un autre but de l'invention est de proposer un gel dont les caractéristiques cosmétiques soient améliorées, et notamment le toucher.

Un autre but encore de l'invention est de proposer un gel à activité physique et à efficacité antimicrobienne qui puisse être incorporé dans la formule du produit cosmétique, dans une proportion inférieure ou égale à 20 %.

La présente invention a pour objet un gel à activité physique antimicrobienne pour produits cosmétiques ou dermopharmaceutiques, contenant au moins un polyol, au moins un polymère de l'acide (meth) acrylique, et un solvant fluidifiant,

caractérisé en ce que le solvant fluidifiant est de formule générale :

$$R1 - 0 - (R2 - 0 - R2-)_n - 0R3 \qquad (1)$$

dans laquelle R1 est un atome d'hydrogène ou une chaîne alkyle en C1 à C5 linéaire ou ramifiée, R2 est une chaîne alkyle en C1 à C5 linéaire ou ramifiée, R3 est un atome d'hydrogène ou une chaîne alkyle en C1 à C5 linéaire ou ramifiée, et n est une valeur entière de 1 à 200.000, en ce que l'un des polyols est la glycérine, et en ce que :

- la proportion en polyol (s) dans le gel est de 1 à 99 %,

- la proportion en polymère(s) dans le gel est de 0,05 à 1 %,

- la proportion en solvant fluidifiant dans le gel est de 5 à 50 %.

Selon d'autres caractéristiques :

- chacun des polyols est de formule générale a, b,... i... - (alkyl) - poly - ol
  dans laquelle :

  · alkyl est une chaîne alkyle, linéaire ou ramifiée en Cn, avec n allant de 2 à 10,

  · poly représente le nombre de fonctions alcool,

  · a, b,...i, représentent des nombres de 1 à 10, différents, correspondant aux positions de substitution des fonctions alcool ;

- de préférence, R1 est une chaîne alkyle en C1 à C3 et n a une valeur de 1 à 3.
- la proportion des solvants fluidifiants dans le gel est de 15 à 30 % ;
- les polymères sont dérivés de l'acide acrylique et/

ou méthacrylique et se présentent sous forme de sels, d'esters, d'amides de ces acides ;

- la proportion des polymères dans le gel est de 0,2 à 1 % ;
- la proportion des polyols dans le gel est de 30 à 70 % ;
- les polyols sont constitués par la glycérine dans la proportion de 30 à 50 % et le 1,2 - octane - diol dans la proportion de 2 à 8 %.
- la proportion du gel dans le produit fini est de préférence de 5 à 20 %.

L'invention a également pour objet l'application du gel précité à la conservation des produits cosmétiques ou dermopharmaceutiques en raison de son activité physique antimicrobienne.

L'invention est décrite ci-après au moyen d'exemples pour les différents composants du gel qui est lui-même constitué de polyols, de polymères du type poly(meth)acrylate, de solvants fluidifiants et d'eau.

Les polyols, dont la formule générale est a, b,... i... (alkyl) - poly - ol peuvent être, à titre d'exemple :

- le propylèneglycol (a = 1, b = 2; n = 3)

- le 1,3 butylèneglycol (a = 1, b = 3, n = 4)

- la glycérine (a = 1, b = 2, c = 3, n = 3)

- le 1,2 pentane diol (a = 1, b = 2, n = 5)

- le 1,2 octane diol (a = 1, b = 2, n = 8)

- le 1,8 octane diol (a = 1, b = 8, n = 8)

- le mannitol ou sorbitol (a = 1, b = 2; c = 3, d = 4, e = 5, f = 6, n = 6)

- le 2 éthyl - 1,3 - hexane dio] (a = 1 , b = 3, n = 8, ramifié).

Les polyols sont choisis en mélange à forte proportion de glycérine. Un mélange particulièrement préféré contient de la glycérine dans une proportion dans le gel de 30 à 50 %, et du 1,2 - octane diol dans une proportion dans le gel de 2 à 8 %.

Ce mélange d'un autre polyol à la glycérine améliore le toucher, la formulabilité et augmente l'effet osmotique du gel c'est-à-dire sa faculté à capter l'eau libre, donc son efficacité anti-microbienne.

Les polymères sont choisis de préférence parmi les sels sodiques, potassiques, triéthylaminiques, triéthanolaminiques, ammoniacaux de l'acide acrylique et/ou de l'acide méthacrylique, mais aussi parmi les esters ou les amides de ces polymères acides, ou les dérivés réticulés du type carbomer - réticulation par des éthers allyliques de pentaérythritol, de sucrose, ou de propylène, par exemple.

La proportion de polymère dans le gel est de référence de 0,2 à 1 %.

Les solvants fluidifiants sont choisis de préférence parmi les éthers comme le méthoxydiglycol, l'éthoxydiglycol (ou diéthylèneglycol monoéthyléther), le propoxydiglycol, le butoxydiglycol, le triéthylèneglycol monopropyléther, ou leurs esters comme l'éthoxydiglycol acétate, ou l'éthoxyéthanol acétate, par exemple. Les solvants fluidifiants peuvent aussi être choisis parmi les polyéthylèneglycols ou les polypropylèneglycols, dont le degré de polymérisation peut aller jusqu'à 200.000. Ces solvants sont avantageusement employés en mélanges.

Un exemple de gel selon l'invention est constitué de :

- 40 % de glycérine et 6 % de 1,2-octanediol,

- 0,7 % de sodium polyacrylate,

- 20 % de éthoxydiglycol.

Le reste, soit 33,3 % est constitué par de l'eau.

Le gel selon l'invention est incorporé au produit fini dans une proportion pouvant aller de 1 à 99 % mais, de préférence, de 5 à 20 %.

Le gel selon l'invention peut être utilisé dans toute forme galénique employée en cosmétique qui nécessite une conservation antimicrobienne : émulsions H/E et E/H, laits, lotions, gels, pommades, lotions capillaires, shampooings et après-shampooings, savons.

Les gels selon l'invention peuvent être combinés dans les compositions cosmétiques avec tout autre ingrédient habituellement utilisé en cosmétique : lipides d'extraction et ou de synthèse, polymères gélifiants et viscosants, tensioactifs et émulsifiants, principes actifs hydro- ou liposolubles, extraits de plantes, extraits tissulaires, extraits marins.

Les compositions cosmétiques contenant les gels selon l'invention peuvent être destinées aux traitements et aux soins de la peau, des cheveux, des ongles et du cuir chevelu, à savoir le traitement anti-vieillissement, antirides, anti-inflammatoire, le traitement de la peau acnéique, la protection solaire et antiradicalaire, l'hydratation et l'effet de lissage, le traitement contre la chute des cheveux, leur protection contre la pollution et l'agresssion.

Il est particulièrement intéressant d'utiliser les gels selon l'invention dans des produits cosmétiques et dermopharmaceutiques destinés aux peaux sensibles, facilement irritées, donc de les incorporer dans les produits dits "hypoallergéniques", "calmants", ou "adoucissants", par exemple : ils ont une propriété anti-irritante.

L'efficacité de ces gels est basée sur un effet physique (osmose). Elle permet de formuler des produits finis qui sont exempts de conservateurs chimiques.

## Revendications

1. Gel à activité physique antimicrobienne pour produits cosmétiques ou dermopharmaceutiques, contenant au moins un polyol, au moins un polymère de l'acide (meth) acrylique, et un solvant fluidifiant, **caractérisé en ce que** le solvant fluidifiant est de formule générale :

$$R1 - 0 - (R2 - 0 - R2\text{-})_n - OR3 \qquad (1)$$

dans laquelle R1 est un atome d'hydrogène ou une chaîne alkyle en C1 à C5 linéaire ou ramifiée, R2 est une chaîne alkyle en C1 à C5 linéaire ou ramifiée, R3 est un atome d'hydrogène ou une chaîne alkyle en C1 à C5 linéaire ou ramifiée, et n est une valeur entière de 1 à 200.000, **en ce que** l'un des polyols est la glycérine,
et **en ce que** :

- la proportion en polyol (s) dans le gel est de 1 à 99 %,

- la proportion en polymère(s) dans le gel est de 0,05 à 1 %,

- la proportion en solvant fluidifiant dans le gel est de 5 à 50 %.

2. Gel selon la revendication 1, **caractérisé en ce que** chacun des polyols est de formule générale a, b,... i... - (alkyl) - poly - ol
dans laquelle :

. alkyl est une chaîne alkyle, linéaire ou ramifiée en Cn, avec n allant de 2 à 10,

. poly représente le nombre de fonctions alcool,

. a, b,...i, représentent des nombres de 1 à 10, différents, correspondant aux positions de substitution des fonctions alcool.

3. Gel selon la revendication 1, **caractérisé en ce que** de préférence, R1 est une chaîne alkyle en C1 à C3 et n a une valeur de 1 à 3.

4. Gel selon la revendication 1, **caractérisé en ce que** la proportion des solvants fluidifiants dans le gel est de 15 à 30 %.

5. Gel selon la revendication 1, **caractérisé en ce que** les polymères sont dérivés de l'acide acrylique et/ ou méthacrylique et se présentent sous forme de sels, d'esters, d'amides de ces acides.

**6.** Gel selon la revendication 1, **caractérisé en ce que** la proportion des polymères dans le gel est de 0,2 à 1 %.

**7.** Gel selon la revendication 1, **caractérisé en ce que** la proportion des polyols dans le gel est de 30 à 70 %.

**8.** Gel selon la revendication 6, **caractérisé en ce que** les polyols sont constitués par la glycérine dans la proportion de 30 à 50 % et le 1,2 - octane - diol dans la proportion de 2 à 8 %.

**9.** Composition cosmétique ou dermopharmaceutique renfermant un gel selon l'une quelconque des revendications 1 à 8 dans une proportion de 5 à 20 %.

**10.** Application du gel selon l'une des revendications 1 à 8 à la conservation des produits cosmétiques ou dermopharmaceutiques en raison de son activité physique antimicrobienne.

**Patentansprüche**

**1.** Gel mit einer physikalischen antimikrobiellen Wirkung für Kosmetikprodukte oder Dermatika, welches mindestens einen mehrwertigen Alkohol (Polyol), mindestens ein Polymer der (Meth)acrylsäure und ein verflüssigendes Lösungsmittel enthält, **dadurch gekennzeichnet, dass** das verflüssigende Lösungsmittel die allgemeine Formel:

$$R1 - O - (R2 - O - R1-)_n - OR3 \qquad (1)$$

aufweist, in der R1 ein Wasserstoffatom oder eine lineare oder verzweigte Alkylkette von C1 bis C5 ist, R 2 eine lineare oder verzweigte Alkylkette von C1 bis C5 ist, R3 ein Wasserstoffatom oder eine lineare oder verzweigte Alkylkette von C1 bis C5 ist und n ein ganzzahliger Wert zwischen 1 und 200.000 ist, und wobei eines der Polyole Glyzerin ist, und dass:

- der Anteil an Polyol (en) in dem Gel zwischen 1 und 99 % liegt,

- der Anteil an Polymer(en) in dem Gel zwischen 0,05 und 1 % liegt,

- der Anteil an verflüssigendem Lösungsmittel in dem Gel zwischen 5 und 50 % liegt.

**2.** Gel nach Anspruch 1, **dadurch gekennzeichnet, dass** jedes der Polyole die allgemeine Formel a, b, ... i ... - (Alkyl) - Poly - ol aufweist, in der:

. Alkyl eine lineare oder verzweigte Alkylkette mit Cn aufweist, wobei n von 2 bis 10 läuft,

. Poly die Zahl der Hydroxylgruppen darstellt,

. a, b, ... i, unterschiedliche Zahlen von 1 bis 10 darstellen, die den Positionen entsprechen, an denen die Hydroxylgruppen substituiert worden sind.

**3.** Gel nach Anspruch 1, **dadurch gekennzeichnet, dass** R1 vorzugsweise eine Alkylkette von C1 bis C3 ist und n einen Wert von 1 bis 3 hat.

**4.** Gel nach Anspruch 1, **dadurch gekennzeichnet, dass** der Anteil der verflüssigenden Lösungsmittel in dem Gel zwischen 15 und 30 % liegt.

**5.** Gel nach Anspruch 1, **dadurch gekennzeichnet, dass** die Polymere von der Acrylsäure und/oder der Methacrylsäure abgeleitet sind und sich in der Form von Salzen, Estern oder Amiden dieser Säuren darstellen.

**6.** Gel nach Anspruch 1, **dadurch gekennzeichnet, dass** der Anteil der Polymere in dem Gel zwischen 0,2 und 1 % liegt.

**7.** Gel nach Anspruch 1, **dadurch gekennzeichnet, dass** der Anteil der Polyole in dem Gel zwischen 30 und 70 % liegt.

**8.** Gel nach Anspruch 6, **dadurch gekennzeichnet, dass** die Polyole aus Glyzerin mit einem Anteil von 30 bis 50 % und 1,2-Oktandiol in einem Anteil von 2 bis 8 % zusammengesetzt sind.

**9.** Kosmetikprodukt oder Dermatikum, welches ein Gel nach einem der Ansprüche 1 bis 8 in einem Anteil von 5 bis 20 % enthält.

**10.** Anwendung eines Gels nach einem der Ansprüche 1 bis 8 zur Konservierung von Kosmetikprodukten oder Dermatika aufgrund seiner physikalisch antimikrobiellen Wirkung.

**Claims**

**1.** Physically active antimicrobial gel for cosmetic or skincare products, containing at least one polyol, at least one polymer of (meth)acrylic acid, and a liquefying solvent
**characterised in that**
the liquefying solvent has the general formula:

$$R1-O-(R2-O-R2)_n-OR3 \qquad (1)$$

in which R2 is a hydrogen atom or a C1 to C5 alkyl chain which may be linear or branched, R2 is a C1 to C5 alkyl chain which may be linear or branched, R3 is a hydrogen atom or a C1 to C5 alkyl chain which may be linear or branched and n is an integer between 1 and 200,000, **in that** one of the polyols is glycerol, and **in that**:

- the proportion of polyol(s) in the gel is from I to 99%,
- the proportion of polymer(s) in the gel is from 0.05 to 1%,
- the proportion of liquefying solvent(s) in the gel is from 5 to 50%.

2. Gel according to Claim 1,
   **characterised in that**
   each of the polyols has the general formula:

   a, b, ... i ... - (alkyl) - poly - ol

   in which:

   - alkyl is a $C_n$-alkyl chain which may be linear or branched, with n from 2 to 10,
   - poly represents the number of alcohol functions,
   - a, b, ... i represent different numbers from 1 to 10 corresponding to the substitution positions of the alcohol functions.

3. Gel according to Claim 1,
   **characterised in that**
   R1 is preferably a C1 to C3-alkyl chain and n has a value from 1 to 3.

4. Gel according to Claim 1,
   **characterised in that**
   the proportion of liquefying solvents in the gel is from 15 to 30%.

5. Gel according to Claim 1,
   **characterised in that**
   the polymers are derived from acrylic and/or methacrylic acid and are in the form of salts, esters or amides of these acids.

6. Gel according to Claim 1,
   **characterised in that**
   the proportion of the polymers in the gel is from 0.2 to 1%.

7. Gel according to Claim 1,
   **characterised in that**
   the proportion of the polyols in the gel is from 30 to 70%.

8. Gel according to Claim 6,
   **characterised in that**
   the polyols consist of glycerol in a proportion of 30 to 50% and 1, 2-octane-diol in a proportion of 2 to 8%.

9. Cosmetic or skincare composition comprising a gel according to any of Claims 1 to 8 in a proportion of 5 to 20%.

10. Application of the gel according to any of Claims I to 8 to the preservation of cosmetic or skincare products by virtue of its physical antimicrobial action.